# EUROPEAN PATENT SPECIFICATION

(11) **EP 1 894 522 B1**
(45) Date of publication and mention of the grant of the patent: **08.04.2009**
(21) Application number: 07001165.5
(22) Date of filing: 19.01.2007
(51) Int. Cl.: A61B 5/04, A61N 1/05, A61B 18/14

(54) **Implantable multielectrode microprobe using shape memory alloy**
Implantierbare Mehrelektrodenmikrosonde unter Verwendung von Formspeicherlegierungen
Microsonde multi-électrodes implantable utilisant un alliage à mémoire de forme

(30) Priority: 29.08.2006 EP 06018000
(43) Date of publication of application: 05.03.2008
(73) Proprietor: Fraunhofer-Gesellschaft zur Förderung der angewandten Forschung e.V., 80686 München (DE)
(72) Inventor: Martinez, Juan, Gómez, Dipl.-Ing., 66386 St. Ingbert (DE); Bossi, Silvia, 00139 Rom (IT); Koch, Klaus Peter, Dr. Ing., 66687 Wadern-Oberlöstern (DE); Micera, Silvestro, 56100 Pisa (IT); Hoffmann, Klaus Peter, Dr., 66386 St. Ingbert (DE)
(74) Representative: Gagel, Roland

(56) References cited:
- WO-A-99/44524
- WO-A-20/06041738
- US-A1- 2003 078 573
- US-A1- 2006 061 348

## Description

### Field of the invention

The present invention relates to an implantable microprobe comprising several electrodes for stimulating and/or recording neural signals.

The stimulation of the cortex and the signal recording from the neural networks have reached a remarkable importance within neuroscience research. The investigations regarding cortex stimulation are focused on the relief of pain, malfunctions caused by strokes, and other disorders including mental illness. The recording of neural signals inside the brain is essential for the study of brain behavior and the design of brain machine interfaces. Implantable microprobes like that of the present invention can be applied for this recording task.

In implantable cortical microprobes the insertion process is a critical point. The probe must be sufficiently strong to pierce the brain tissue without damaging itself. During the insertion, the pia matter which covers the cortex has to be pierced first. This layer is 60 µm thick and more than 15 times stiffer than the cortex (2.7 to 3.5 mm thick). On the other hand, for long-term stimulation/measurements the microprobe must be sufficient flexible to withstand the inner micromotions without fracturing or damaging the cortical tissue.

### Background of the invention

Different microprobes for implantation into live tissue have been described in the last years. US 2003/0100823 A1 discloses an implantable microprobe comprising several electrodes and electrical junctions for electrically contacting the electrodes at one end of the microprobe. The electrodes are embedded in a polyimid encapsulation allowing a high flexibility of the microprobe after implantation. Since this polyimid microprobe is not strong enough to pierce the brain tissue, however, pre-incisions have to be made with stiff instruments prior to the insertion of the microprobe.

Such pre-incisions are not necessary when using stiff microprobes as disclosed for example in A. B. Frazier et al., "Two-Dimensional Metallic Microelectrode Arrays for Extracellular Stimulation and Recording of Neurons", PIEEE Proc. Micro. Electro. Mech. Syst., pp. 195 to 200, 1993. The array of electrodes in this microprobe is fabricated on a silicon substrate using nickel as the structural material. Due to this metal base the microprobe has enough stiffness to pierce the brain tissue without bending or buckling. On the other hand, however, such a stiff microprobe lacks the necessary flexibility at the implanted position.

To avoid the above mentioned problems WO 2004/009176 A1 proposes an implantable microprobe with a flexible region to accommodate micro-movement inside the tissue. The microprobe is formed of two silicon substrate portions which are connected by a flexible portion of a BCB or polyimid material. The two silicon substrate portions form a rigid backbone when inserting the microprobe, whereas the flexible portion provides flexibility and absorbs stress from any relative movement of brain tissue and outside forces after implantation.

US 2003/0078573 discloses an electrosurgical working end for controlled energy delivery in a targeted tissue volume, e.g. for thermally-medicated treatments or ablations of tumors or other targeted tissues. The working end comprises at least one resilient or flexible element of a shape memory alloy extending from a first end along a longitudinal axis towards a second end of said working end, several electrodes and electrical junctions for electrically contacting said electrodes and said element at the second end. The element of shape memory alloy, which forms one of the electrodes for applying RF energy to the tissue, is embedded in a flexible polymer encapsulation.

It is an object of the present invention to provide a further implantable microprobe which is stiff enough for piercing the brain tissue and flexible enough to withstand the inner micro-motions after implantation without fracturing or damaging the cortical tissue.

### Summary of the invention

The object is achieved with the implantable microprobe according to claim 1. Advantageous embodiments of this microprobe are subject matter of the subclaims or are described in the following description and/or exemplary embodiments of the invention.

The proposed implantable microprobe comprises at least one element of a shape memory alloy extending from a first end along a longitudinal axis towards a second end of said microprobe and several electrodes, said element and said electrodes being embedded in a flexible polymer encapsulation. At the second end of the microprobe electrical junctions are arranged for electrically contacting said electrodes and said element. The element of shape memory alloy is designed to be straight and stiff at a higher temperature and more flexible at lower temperatures.

In this implantable microprobe the element of shape memory alloy forms a backbone which changes its mechanical properties and optionally also its form with the temperature. Therefore, by thermal activation of the element the mechanical properties and optionally also the shape of the microprobe changes due to the element. During the insertion process, the element is heated to a sufficiently high temperature. Therefore, the Young's modulus of the microprobe highly increases and the microprobe becomes completely stiff and straight for an effective insertion. Then, when the probe is brought inside the cortex, the shape memory alloy is inactivated and the source of temperature removed. Therefore, the probe preferably returns to a shape for a better recording and stimulation and its Young's modulus decreases, making the probe more flexible inside the cortical tissue. The shape memory alloy element ensures that the implantable microprobe is stiff enough to be inserted into the cerebral cortex and changes its mechanical properties to be flexible inside of the cortical tissue or locates with in a desired position.

The element of shape memory alloy is formed such that it has a straight form at the higher temperatures and a desired form, for example a curved form, at lower temperatures, which is may be more appropriate for the desired recording and/or stimulation.

The element of shape memory alloy preferably has the form of a rod or a needle at the higher temperature. Furthermore, this rod or needle preferably extends through the encapsulation at the first end of the microprobe to form a peak at this end. This peak helps to perform a micro-incision on the pia matter with the implantable microprobe. Subsequently the insertion process is carried out.

The heating of the element of shape memory alloy is preferably performed by applying an AC or DC current to the element through the electrical junctions. The temperature can be controlled by an appropriate temperature control unit to maintain the required temperature (for the stiffness of the element) during the piercing and insertion process. It is obvious that the dimensions of the element must be selected such that a sufficient stiffness of the microprobe is achieved for the desired piercing and insertion process.

The insertion process can also be performed using electrosurgical principles of cell vaporisation, which avoids the coagulation of the tissue during insertion. To this end, in an embodiment in which the element extends through the encapsulation at the first end, an AC voltage is applied to this element for heating. Due to the contact of the element with the tissue and the applied AC voltage, the desired cell vaporisation is achieved.

The proposed implantable microprobe preferably has an elongated flat structure with a tip at the first end. The element of shape memory alloy preferably extends from this first end with the tip to the second end. This element is isolated through the polymer encapsulation from the embedded electrodes which are arranged for direct contact with the tissue. The electrodes are preferably made of platinum and connected through appropriate wires to the electrical junctions. Nevertheless, also other materials like gold can be used for the electrodes. The shape memory alloy preferably is made of Nitinol. However, also other types of shape memory alloys can be used which allow the higher stiffness at reasonable higher temperatures which do not destroy the polymer encapsulation.

The proposed implantable multielectrode microprobe has the capability of changing its mechanical properties to improve the insertion process and the long term implantation. It is a small, portable and easy to manage device for insertion into a live tissue.

### Short description of the drawings

The proposed microprobe is described in the following by way of examples in connection of the accompanying figures without limiting the scope of protection as defined by the claims. The figures show:
- Fig. 1: an example for an application of the proposed microprobe;
- Fig. 2: a view showing an example of the proposed microprobe;
- Fig. 3: a cut through the microprobe of Fig. 2;
- Fig. 4: an example of a substrate for connecting the microprobe;
- Fig. 5: an example of a tool for performing the insertion process with the microprobe;
- Fig. 6: a top view of the tool of figure 5;
- Fig. 7: an example of a complete tool for inserting the microprobe; and
- Fig 8: a view showing a further example of the form of the shape memory alloy in the proposed microprobe.

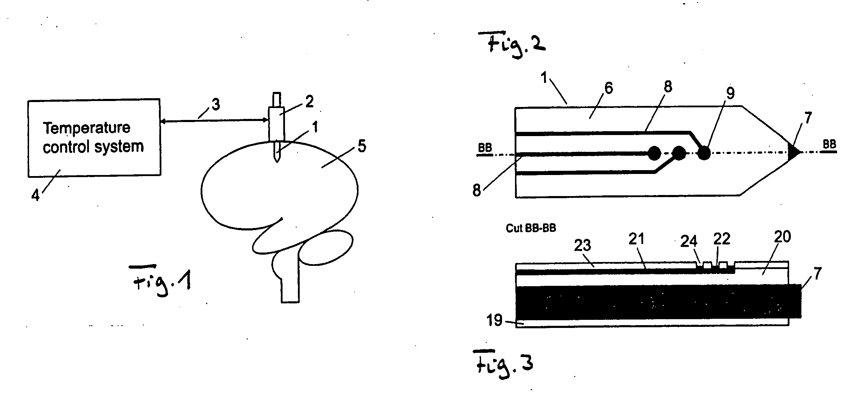

Figure 1 shows an example for an application of the proposed implantable microprobe. In this example the microprobe 1 is inserted with a special tool 2 in the target tissue 5 of a brain. To this end the tool 2 is placed perpendicular to the target tissue 5. The microprobe 1 is placed in an insertion opening of the tool 2 to perform the penetration into the tissue. During this insertion process the temperature of the element of shape memory alloy inside the microprobe 1 is controlled by a temperature control system 4 connected to the microprobe 1 via an appropriate connection 3.

Figure 2 depicts an exemplary embodiment of the proposed microprobe 1. The microprobe 1 consists of several platinum electrodes 9, connected with gold wires or tracks 8 to outside, and a shape memory alloy (SMA) needle 7 embedded in a polymer encapsulation 6. The gold tracks 8 form electrical junctions at the second end of the microprobe 1 to ensure that the platinum electrodes 9 can be electrically connected via the gold tracks 8 from outside of the microprobe. The polymer encapsulation 6 forms a tip at the first end of the microprobe 1. At this end, the encapsulated SMA needle 7 extends through this encapsulation and forms a peak as shown in Figure 2. The SMA needle 7 extends along the longitudinal axis of the microprobe 1 throughout the whole length of this microprobe to the second end as can be seen in Figure 3.

Figure 3 shows a cut through the line BB-BB of Figure 2. The microprobe 1 is manufactured on a silicon wafer (not shown in the figures). A first layer of a spinning polymer 19 is placed on the silicon wafer. Then, the shape memory alloy 7 is deposited over the spinning polymer layer 19. A second polymer layer 20 is spun over the shape memory alloy 7. A thin layer of gold 21 is sputtered on the polymer layer 20 to form the tracks 8. A thin film layer of platinum 22 is sputtered on the polymer layer 20 to form the electrodes 9. Finally, a third layer of a spinning polymer 23 is placed over the gold 21 and platinum 22 layers to form the isolation. Several vias or holes 24 are made over the platinum layer 22 to form the electrodes and leave them in contact with the tissue. The structuring of the gold tracks 8, the electrodes 9, the holes 24 and the SMA needle 7 can be performed with known photolithographic processes.

Prior to the encapsulation, SMA needle 7 is processed to obtain the desired mechanical behavior. The objective is to obtain a completely straight and stiff SMA needle 7 when inserting the microprobe into the live tissue, and a more flexible one once the microprobe is inside the tissue. During the memorization process for this objective, the needle 7 is heated up with the straight shape during 35 minutes at a temperature of 450°C. Then, a rapid cooling of the SMA is performed. Once this treatment is finished, the straight shape is obtained when the SMA reaches a temperature of 69°C (austenite state) by applying a DC or an AC current source. These values apply to the use of Nitinol as the shape memory alloy. As commonly known, other temperatures are necessary with other types of shape memory alloys. To maintain the straight shape and stiffness of the SMA needle 7 it is necessary to keep the above temperature by using temperature control system 4 (see Fig. 1). During the austenite state the Young's modulus of the SMA increases, reaching 75 GPa. The dimensions of the SMA needle 7 and the microprobe are calculated to be able to pierce the live tissue without bending and breaking when using the insertion tool 2. Once the microprobe 1 is inside the live tissue, the current source is removed and the temperature of the SMA needle 7 decreases. The SMA then reaches the martensite state and reduces its Young's modulus to 28 GPa, making the microprobe more flexible inside the tissue.

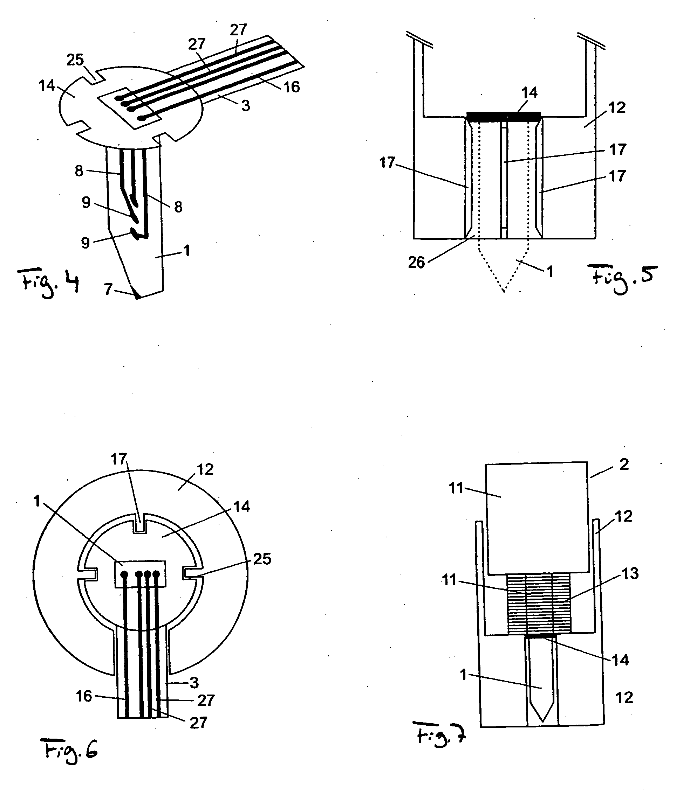

To perform the insertion process, insertion tool 2 is used in the present example. The electrical connection of the microprobe 1 is made using a silicon substrate 14, which is attached to the microprobe 1 as shown in Figure 4. This silicon substrate 14 comprises electrical junctions matching the electrical junctions of the microprobe 1. Through this electrical junctions the tracks 8 of the microprobe 1 are electrically connected to the wires 27 of the substrate 14 in order to be able to stimulate or record from outside, i.e. from an appropriate recording and/or stimulation unit (not shown). The SMA needle 7 is also connected via wire 16 through the substrate 14. The second electrical contact required for electrically heating the SMA needle 7 can be provided at the tip of the needle. The wires 16 and 27 are encapsulated with a polymer outside of the substrate 14 to form a connection 3 to the temperature control system 4 including the AC or DC current source and to the recording and/or stimulation unit.

Insertion tool 2 has a guidance opening or (hollow 26) for insertion of the microprobe 1 (see Fig. 5). Form and dimensions of this hollow 26 are such that the silicon substrate 14 connected to the microprobe exactly matches inside this hollow as can be seen in the top view of Figure 5. To this end appropriate guides 17 are formed within the hollow 26 to mate with appropriate recesses 25 in the silicon substrate 14.

Microprobe 1 is connected to the silicon substrate 14, allowing the microprobe to slide through hollow 26 of the insertion tool 2. At the upper side, insertion tool 2 has a guidance opening for a piston 11, through which a pressure can be applied to the substrate 14 and the microprobe 1 through a spring 13 (see Fig. 7). Applying the pressure with piston 11 the silicon substrate 14 slides, guided by the guides 17, through hollow 26 penetrating the microprobe 1 into the live tissue. Prior to this insertion the SMA needle 7 is heated up through the wire 16 to the appropriate temperature, i.e. 69 °C in case of Nitinol.

In this example, SMA needle 7 embedded into microprobe 1 is also used as an electro scalpel like tool. During the procedure for the insertion into the motor cortex, microprobe 1 is placed in non direct contact above the pia matter. Applying an alternating current through wire 16 to SMA needle 7 in order to increase its temperature the pia matter is cut by vaporising the tissue getting in contact with the peak of SMA needle 7 with the applied AC current. The heating process is controlled by the temperature control system 4. Once the microlesion is made, the applied current is removed and the insertion into the cortex is performed.

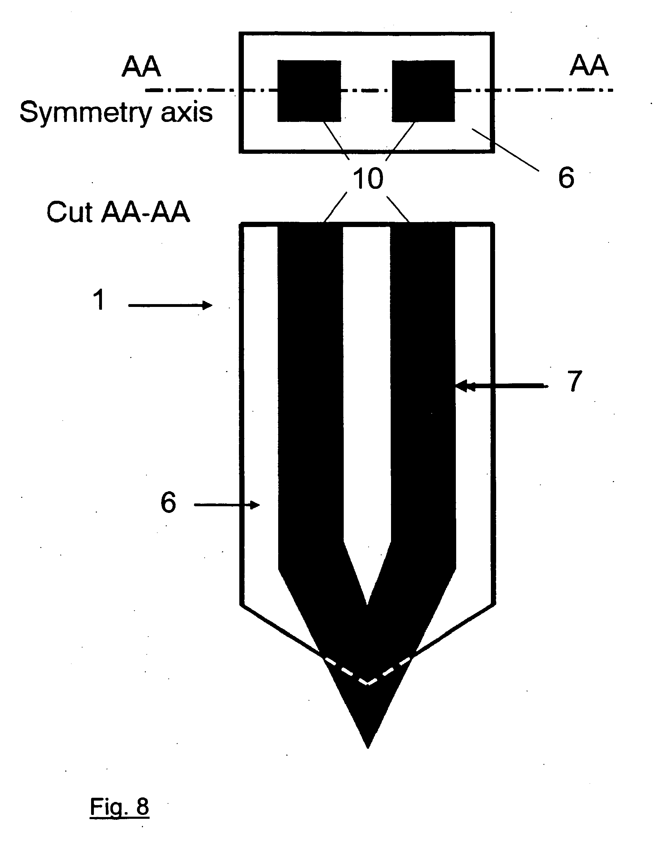

Figure 8 shows a further exemplary embodiment of the form of the shape memory alloy 7 (SMA) of the proposed microprobe 1 in two different cutting planes. In the figure only shape memory alloy 7 is depicted for simplicity. The electrodes and tracks or wires are omitted. SMA 7 is formed of two separate legs 10 embedded in polymer encapsulation 6. The legs 10 are joint at the first end of the micro probe 1. At this end, the encapsulated SMA 7 forms a peak which extends through this encapsulation as shown in Figure 8. The temperature of the SMA 7 can be controlled by applying a direct or alternating current through the two legs 10 of the SMA 7, For applying the heating current these legs 10 can be electrically contacted easily at the second end of the microprobe 1.

While the invention has been illustrated and described in detail in the drawings and foregoing description, such illustration and description are to be considered illustrative or exemplary and not restrictive; the invention is not limited to the disclosed embodiments. For example, it is also possible to provide more or less than the three electrodes shown in the figures. The SMA element can have another straight form. The electrical connection to the microprobe can be realized in another manner than through the substrate shown. Furthermore, the tool for inserting the microprobe can be different from the tool shown in the figures.

### List of reference signs

- 1: microprobe
- 2: insertion tool
- 3: connection
- 4: temperature control system
- 5: brain tissue
- 6: polymer encapsulation
- 7: SMA needle
- 8: gold tracks
- 9: electrodes
- 10: legs of SMA
- 11: piston
- 13: spring
- 14: silicon substrate
- 16: connecting wire
- 17: guides
- 19: polymer layer
- 20: polymer layer
- 21: gold layer
- 22: platinum layer
- 23: polymer layer
- 24: holes
- 25: recess
- 26: hollow
- 27: connecting wires

## Claims

1. Implantable microprobe comprising
at least one element (7) of a shape memory alloy extending from a first end along a longitudinal axis towards a second end of said microprobe (1), several electrodes (9) and electrical junctions for electrically contacting said electrodes (9) and said element (7) at the second end of the microprobe (1),
wherein said element (7) and said electrodes (9) are embedded in a polymer encapsulation (6) and wherein said element (7) is designed to be stiff at a higher temperature and more flexible at lower temperatures,
**characterized in that** said element (7) is designed to be straight at the higher temperature.

2. Implantable microprobe according to claim 1, wherein said element (7) has the form of a rod or a needle.

3. Implantable microprobe according to claim 1, wherein said element (7) is formed of two legs (10) joint at the first end.

4. Implantable microprobe according to one of claims 1 to 3, wherein said element (7) forms a peak at the first end of the microprobe (1).

5. Implantable microprobe according to one of claims 1 to 4, wherein said polymer encapsulation (6) is a polyimid encapsulation.

6. Implantable microprobe according to one of claims 1 to 5, wherein said shape memory alloy is Nitinol.

7. Implantable microprobe according to on of claims 1 to 6, wherein said element (7) is connected to a temperature control system (4), applying a DC or AC current to achieve and maintain the higher temperature of the element (7) during a period of time.

## Patentansprüche

1. Implantierbare Mikrosonde, umfassend
zumindest ein Element (7) aus einer Formgedächtnislegierung, das sich von einem ersten Ende entlang einer Längsachse zu einem zweiten Ende der Mikrosonde (1) hin erstreckt,
mehrere Elektroden (9) und elektrische Verbindungen zum elektrischen Kontaktieren der Elektroden (9) und des Elements (7) am zweiten Ende der Mikrosonde (1),
wobei das Element (7) und die Elektroden (9) in einer Polymerverkapselung (6) eingebettet sind und wobei das Element (7) dazu ausgelegt ist, bei einer höheren Temperatur steif zu sein und bei niedrigeren Temperaturen biegsamer zu sein,
**dadurch gekennzeichnet, dass** das Element (7) dazu ausgelegt ist, bei der höheren Temperatur gerade zu sein.

2. Implantierbare Mikrosonde nach Anspruch 1, wobei das Element (7) die Form eines Stabs oder einer Nadel aufweist.

3. Implantierbare Mikrosonde nach Anspruch 1, wobei das Element (7) aus zwei Gliedern (10) gebildet ist, die am ersten Ende verbunden sind.

4. Implantierbare Mikrosonde nach einem der Ansprüche 1 bis 3, wobei das Element (7) am ersten Ende der Mikrosonde (1) eine Spitze bildet.

5. Implantierbare Mikrosonde nach einem der Ansprüche 1 bis 4, wobei die Polymerverkapselung (6) eine Polyimidverkapselung ist.

6. Implantierbare Mikrosonde nach einem der Ansprüche 1 bis 5, wobei die Formgedächtnislegierung Nitinol ist.

7. Implantierbare Mikrosonde nach einem der Ansprüche 1 bis 6, wobei das Element (7) mit einem Temperaturregelsystem (4) verbunden ist, das einen Gleich- oder Wechselstrom anlegt, um die höhere Temperatur des Elements (7) während eines Zeitraums zu erzielen und beizubehalten.

## Revendications

1. Microsonde implantable comprenant
au moins un élément (7) en alliage à mémoire de forme qui s'étend d'une première extrémité le long d'un axe longitudinal vers une seconde extrémité de ladite microsonde (1),
plusieurs électrodes (9) et jonctions électriques destinées à établir un contact électrique entre lesdites électrodes (9) et ledit élément (7) au niveau de la deuxième extrémité de la microsonde (1),
ledit élément (7) et lesdites électrodes (9) étant intégrés dans une encapsulation polymère (6) et ledit élément (7) étant conçu pour être rigide à une température supérieure et plus flexible à des températures inférieures,
**caractérisée en ce que** ledit élément (7) est conçu pour être droit à la température supérieure.

2. Microsonde implantable selon la revendication 1, dans laquelle ledit élément (7) a la forme d'une tige ou d'une aiguille.

3. Microsonde implantable selon la revendication 1, dans laquelle ledit élément (7) est formé par deux jambes (10) jointes au niveau de la première extrémité.

4. Microsonde implantable selon l'une quelconque des revendications 1 à 3, dans laquelle ledit élément (7) forme un pic au niveau de la première extrémité de la microsonde (1).

5. Microsonde implantable selon l'une quelconque des revendications 1 à 4, dans laquelle ladite encapsulation polymère (6) est une encapsulation en polyimide.

6. Microsonde implantable selon l'une quelconque des revendications 1 à 5, dans laquelle ledit alliage à mémoire de forme est le Nitinol.

7. Microsonde implantable selon l'une quelconque des revendications 1 à 6, dans laquelle ledit élément (7) est connecté à un système de contrôle de la température (4), appliquant un courant continu ou un courant alternatif pour atteindre et maintenir la température supérieure de l'élément (7) durant une période de temps.
